(19) Europäisches Patentamt
European Patent Office
Office européen des brevets

(11) EP 0 600 294 B1

(12) **EUROPÄISCHE PATENTSCHRIFT**

(45) Veröffentlichungstag und Bekanntmachung des Hinweises auf die Patenterteilung:
**19.02.2003 Patentblatt 2003/08**

(51) Int Cl.[7]: **C08G 65/32**, B01F 17/42, C07C 233/90, C07C 235/88, C07C 307/02, C07C 311/51

(21) Anmeldenummer: **93118470.9**

(22) Anmeldetag: **15.11.1993**

(54) **Polyethersubstituierte Imidverbindungen sowie deren Verwendung**
Polyether substituted imide compounds and their use
Composés imides substitués par une chaîne polyéther et leur utilisation

(84) Benannte Vertragsstaaten:
**BE CH DE ES FR GB IT LI NL**

(30) Priorität: **27.11.1992 DE 4240008**

(43) Veröffentlichungstag der Anmeldung:
**08.06.1994 Patentblatt 1994/23**

(73) Patentinhaber: **BAYER AG**
**51368 Leverkusen (DE)**

(72) Erfinder:
- **Pohmer, Klaus, Dr.**
  **D-51061 Köln (DE)**
- **Weber, Rainer, Dr.**
  **D-51519 Odenthal (DE)**
- **Dörzbach-Lange, Cornelia, Dr.**
  **D-51515 Kürten-Bechen (DE)**
- **Haida, Reinhard**
  **D-51467 Bergisch Gladbach (DE)**
- **Moretto, Hans-Heinrich, Dr.**
  **D-51375 Leverkusen (DE)**

(56) Entgegenhaltungen:
**EP-A- 0 043 108**
**EP-A- 0 096 629**
**EP-A- 0 366 483**
**DE-A- 2 118 241**
**DE-B- 1 140 188**
**FR-A- 2 194 739**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 600 294 B1

Printed by Jouve, 75001 PARIS (FR)

## Beschreibung

Die vorliegende Erfindung betrifft neue polyethersubstituierte Verbindungen von Imiden sowie deren Verwendung als grenzflächenaktive Mittel.

Perfluoralkylgruppenhaltige Polyethertenside finden aufgrund ihrer hohen Grenzflächenaktivität vielfältige Verwendungen in der Technik. Typische Anwendungen sind die Verbesserung von Verlaufs- und Netzeigenschaften bei Lacken und Dispersionsklebern oder in Spül- und Reinigungsmitteln (vergL H.G. Klein, J.N. Meußdoerffer und K. Niederprüm, Metalloberfläche 29 (1975) 559 bis 567). Beispiele für solche Verbindungen sind:

$$C_8F_{17}SO_2N(CH_3)CO\text{-}(O\text{-}CH_2\text{-}CH_2)_{5,67}\text{-}OC_4H_9$$

$$C_8F_{17}SO_2N(C_2H_5)\text{-}(O\text{-}CH_2\text{-}CH_2)_5\text{-}OH$$

$$C_8F_{17}SO_2N(CH_3)CO\text{-}(O\text{-}CH_2\text{-}CH_2)_{10,3}\text{-}OC_4H_9$$

$$C_8F_{17}SO_2N(C_2H_5)\text{-}(O\text{-}CH_2\text{-}CH_2)_{11\text{-}14}\text{-}OH$$

$$C_8F_{17}SO_2N(CH_3)CO\text{-}(O\text{-}CH_2\text{-}CH_2)_{19,5}\text{-}OC_4H_9$$

$$C_8F_{17}SO_2N(CH_3)CO\text{-}(O\text{-}CH_2\text{-}CH_2)_{14}\text{-}(OCH[CH_3]\text{-}CH_2)_{14}\text{-}OC_4H_9$$

Wege zur Synthese der o.g. Verbindungen sind unter anderem in DE-B 1 140 188 und im Artikel von H.G. Klein, J.N. Meußdoerffer und K. Niederprüm in Metalloberfläche 29 (1975) 559 bis 567 beschrieben.

Die Herstellung der perfluorierten Ausgangsverbindungen, aus denen obengenannte Verbindungen hergestellt werden, erfolgt nach drei unterschiedlichen Synthesewegen:

a) elektrochemische Fluorierung,
b) Telomerisation von Perfluorolefinen, insbesondere Tetrafluorethylen,
c) Oligomerisierung von Tetrafluorethylen.

Da die genannten Methoden zur Herstellung der perfluorierten Ausgangsverbindungen technisch sehr aufwendig sind, resultieren hohe Kosten bei der Herstellung der gewünschten perfluorgruppenhaltigen chemischen Verbindungen.

Die Herstellung der obengenannten perfluorgruppenhaltigen Polyethertenside erfolgt üblicherweise durch mehrstufige Synthese über die Reaktion von Perfluoralkylsulfonamiden mit

a) phosgenierten Polyethern oder
b) Ethylen- bzw. Propylenoxid.

Solche Verfahren sind beispielsweise in "Ullmann, Enzyklopädie der technischen Chemie, 4. Aulage 1982, Band 22, Seiten 455 bis 515" und in H.G. Klein, J.N. Meußdoerffer, H. Niederprüm und M. Wechsberg, Metalloberfläche 29 (1975) 559 bis 567, beschrieben. Nachteil dieser Verfahren ist,daß sie sehr aufwendig sind.

Aufgabe war es daher, fluorgruppenhaltige Polyethertenside zur Verfügung zu stellen, die als grenzflächenaktive Mittel eingesetzt werden können und deren Herstellung einfach und kostengünstig möglich ist.

Diese Aufgabe konnte durch die erfindungsgemäßen polyethersubstituierten Imidverbindungen gelöst werden.

Gegenstand der Erfindung sind fluoralkyl- und/oder fluorarylgruppenhaltige Imidverbindungen der allgemeinen Formel (I).

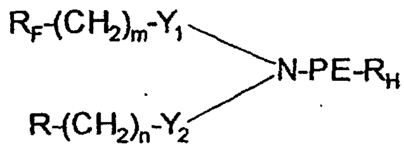 (I) ,

wobei

$R_F$ ein linearer oder verzweigter Fluoralkylrest mit 1 bis 18 Kohlenstoffatomen, ein Fluorarylrest mit 6 bis 12 Kohlenstoffatomen, ein gemischter Fluoralkylarylrest mit 7 bis 18 Kohlenstoffatomen oder ein fluorierter Mono- oder Polyether mit 2 bis 18 Kohlenstoffatomen ist,

R ein linearer oder verzweigter Alkylrest mit 1 bis 24 Kohlenstoffatomen, ein Arylrest mit 6 bis 12 Kohlenstoffatomen oder ein gemischter Alkylarylrest mit 7 bis 24 Kohlenstoffatomen, wobei die Kohlenstoffkette auch durch Sauerstoff-, Stickstoff- oder Schwefelatome unterbrochen sein kann, oder ein weiterer wie oben definierter Rest $R_F$ ist, wobei die beiden $R_F$-Reste gleich oder unterschiedlich sein können,

$Y_1$ und $Y_2$ unabhängig voneinander eine

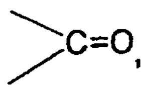, 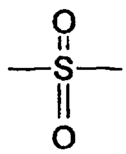, 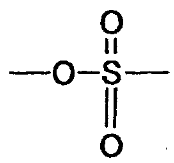 oder 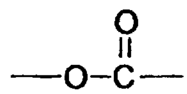 Gruppe

darstellen,

m eine ganze Zahl zwischen 0 und 6 darstellt,

n eine ganze Zahl zwischen 0 und 6 darstellt und

PE eine Polyetherkette bestehend aus 5 bis 50 Ethylenoxid- oder 5 bis 50 Propylenoxid- oder einem Gemisch aus 5 bis 50 Ethylenoxid- und Propylenoxideinheiten darstellt und

$R_H$ ein Wasserstoffatom oder ein linearer oder verzweigter Alkylrest mit 1 bis 10 Kohlenstoffatomen ist.

Fluoralkyl- und/oder fluorarylgruppenhaltige Imidverbindungen sind vorzugsweise solche, bei denen $R_F$ ein linearer oder verzweigter Fluoralkylrest mit 3 bis 10 Kohlenstoffatomen bzw. ein Fluorarylrest mit 6 bis 12 Kohlenstoffatomen ist.

Bevorzugt sind solche fluoralkyl- und/oder fluorarylgruppenhaltigen Imidverbindungen, bei denen $R_F$ für einen linearen oder verzweigten Perfluoralkylrest mit 3 bis 10 Kohlenstoffatomen bzw. für einen Perfluorarylrest mit 6 bis 12 Kohlenstoffatomen steht.

Besonders bevorzugt sind Imidverbindungen, bei denen R für einen linearen oder verzweigten Alkylrest mit 6 bis 14 Kohlenstoffatomen, einen Arylrest mit 6 bis 12 Kohlenstoffatomen, einen gemischten Alkylarylrest mit 7 bis 14 Kohlenstoffatomen oder einen linearen oder verzweigten Perfluoralkylrest mit 3 bis 10 Kohlenstoffatomen steht.

Besonders bevorzugt sind Imidverbindungen, bei denen $Y_1$ und $Y_2$ unabhängig voneinander für eine

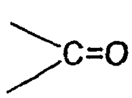 oder 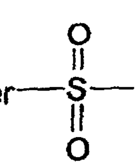 Gruppe stehen.

Imidverbindungen, in denen m und n 0 sind, sind besonders bevorzugt.

Als Endgruppen $R_H$ werden bevorzugt lineare Alkylreste mit 2 bis 4 Kohlenstoffatomen eingesetzt.

Besonders bevorzugte Imidverbindungen weisen z.B. folgende Strukturen auf:

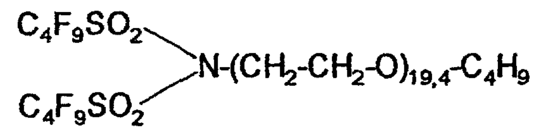

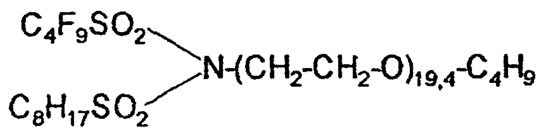

Die erfindungsgemäßen Imidverbindungen lassen sich durch Umsetzung von aminierten Polyethern mit

- Fluorcarbonsäuren
- Fluorsulfonsäuren
- Fluorcarbon- oder Fluorsulfonsäurederivaten

und gegebenenfalls mit

- Carbonsäuren
- Sulfonsäuren
- Carbon- oder Sulfonsäurederivaten

herstellen.

Im folgenden ist ein möglicher Syntheseweg beispielhaft wiedergegeben:

$$R_F\text{-}(CH_2)_m\text{-}Y_1\text{-}A_1 + H_2N\text{-}PE\text{-}R_H + N(CH_2CH_3)_3 \rightarrow$$

$$RF\text{-}(CH_2)_m\text{-}Y_1\text{-}N(H)\text{-}PE\text{-}R_H + [HN(CH_2CH_3)_3]A_1$$

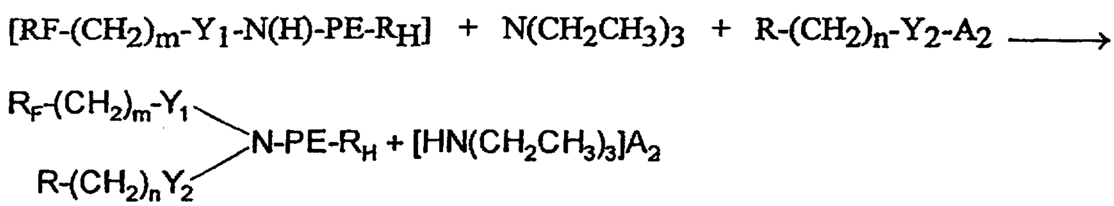

wobei

| | |
|---|---|
| $R_F$, R, $Y_1$, $Y_2$, m, n, PE und $R_H$ | dieselbe Bedeutung wie oben haben und |
| $A_1$ und $A_2$ | unabhängig voneinander reaktive Abgangsgruppen, wie z.B. ein Halogenatom, eine Hydroxy-, eine Alkoxy- oder eine Carboxygruppe, sind. |

Für das vorgenannte Verfahren können folgende Ausgangsverbindungen eingesetzt werden:

Beispiele für Fluorcarbonsäuren:

| | |
|---|---|
| Perfluorheptansäure | $CF_3-(CF_2)_5-COOH$ |
| Perfluoroctansäure | $CF_3-(CF_2)_6-COOH$ |
| Perfluornonansäure | $CF_3-(CF_2)_7-COOH$ |
| Perfluorethercarbonsäure dimer | $CF_3-(CF_2)_2-O-CF(CF_3)-COOH$ |
| Penfluorethercarbonsäure trimer | $CF_3-CF_2-[CF_2-O-CF(CF_3)]_2-COOH$ |
| Perfluorethercarbonsäure tetramer | $CF_3-CF_2-[CF_2-O-CF(CF_3)]_3-COOH$ |
| Perfluorbenzoesäure | $C_6F_5-COOH$ |
| 2,2,3,3,4,4,5,5,6,6,7,7-Dodecafluorheptansäure | $H(CF_2)_6-COOH$ |
| 4,4.5,5,6,6,7,7,8,8,9,9,9-Tridecafluornonansäure | $CF_3-(CF_2)_5(CH_2)_2-COOH$ |
| 4,4,5,5,6,6,7,7,8,8,9,9,10,10,11,11,11-Heptadecafluorundecansäure | $CF_3-(CF_2)_7(CH_2)_2COOH$ |
| 2-Tetrafluorethoxyethansäure | $H(CF_2)_2-O-CH_2-COOH$ |
| 2-Hexafluorpropoxyethansäure | $CF_3-CHF-CF_2-O-CH_2-COOH$ |

Beispiele für Fluorsulfonsäuren:

| | |
|---|---|
| Perfluorbutansulfonsäure | $CF_3-(CF_2)_3-SO_3H$ |
| Perfluorhexansulfonsäure | $CF_3-(CF_2)_5-SO_3H$ |
| Perfluoroctansulfonsäure | $CF_3-(CF_2)_7-SO_3H$ |
| Perfluorbenzolsulfonsäure | $C_6F_5-SO_3H$ |
| Perfluortoluolsulfonsäure | $CF_3-C_6F_4-SO_3H$ |

Beispiele für Fluorsulfon- oder Fluorcarbonsäurederivate:

| | |
|---|---|
| Perfluorbutansäureanhydrid | $[CF_3(CF_2)_2CO]_2O$ |
| Perfluorbutansäurechlorid | $CF_3(CF_2)_2COCl$ |
| Perfluorbutansäureethylester | $CF_3(CF_2)_2COOC_2H_5$ |
| Perfluorbutansulfonsäurefluorid | $CF_3(CF_2)_3SO_2F$ |
| Perfluorhexansulfonsäurefluorid | $CF_3(CF_2)_5SO_2F$ |
| Perfluoroctansulfonsäure | $CF_3(CF_2)_7SO_2F$ |
| Perfluorbenzoesäurechlorid | $C_6F_5COCl$ |
| Perfluorbenzolsulfonsäurechlorid | $C_6F_5SO_2Cl$ |

Beispiele für Carbonsäuren:

| | |
|---|---|
| n-Butansäure | $CH_3CH_2CH_2COOH$ |
| n-Pentansäure | $CH_3(CH_2)_3COOH$ |
| n-Hexansäure | $CH_3(CH_2)_4COOH$ |
| n-Heptansäure | $CH_3(CH_2)_5COOH$ |
| n-Octansäure | $CH_3(CH_2)_6COOH$ |
| n-Nonansäure | $CH_3(CH_2)_7COOH$ |
| n-Decansäure | $CH_3(CH_2)_8COOH$ |
| n-Undecansäure | $CH_3(CH_2)_9COOH$ |
| n-Dodecansäure | $CH_3(CH_2)_{10}COOH$ |

(fortgesetzt)

| | |
|---|---|
| 2-Methylpropansäure | $(CH_3)_2CH-COOH$ |
| 3-Methylbutansäure | $(CH_3)_2CH-CH_2COOH$ |
| 2,2-Dimethylpropansäure | $(CH_3)_3C-COOH$ |
| 2-Methylbutansäure | $CH_3CH_2CH(CH_3)-COOH$ |
| 2-Ethylbutansäure | $CH_3CH_2CH(C_2H_5)-COOH$ |
| 2-Ethylhexansäure | $CH_3(CH_2)_3CH(C_2H_5)-COOH$ |
| isomere $C_8$-Säuren | $C_7H_{15}COOH$ |
| isomere $C_9$-Säuren | $C_8H_{17}COOH$ |
| isomere $C_{13}$-Säuren | $C_{12}H_{25}COOH$ |
| Nonadecansäure | $C_{18}H_{37}COOH$ |
| Cyclohexancarbonsäure | $C_6H_{11}COOH$ |
| Propensäure | $CH_2=CH-COOH$ |
| 2-Methylpropensäure | $CH_2=C(CH_3)-COOH$ |
| trans-3-Methylpropensäure | $CH_3CH=CH-COOH$ |
| cis-3-Methylpropensäure | $CH_3CH=CH-COOH$ |
| 2,3-Dimethylpropensäure | $CH_3CH=C(CH_3)-COOH$ |
| Hexandiensäure | $CH_3CH=CHCH=CH-COOH$ |
| 11-Undecensäure | $CH_2=CH(CH_2)_8COOH$ |
| Acetylencarbonsäure | $CH\equiv C-COOH$ |
| Benzoesäure | $C_6H_5-COOH$ |
| Methylbenzoesäure | $CH_3C_6H_4-COOH$ |
| Phenylessigsäure | $C_6H_5CH_2-COOH$ |
| Naphthylessigsäure | $C_{10}H_7-CH_2-COOH$ |

Beispiele für Sulfonsäuren:

| | |
|---|---|
| Methansulfonsäure | $CH_3SO_3H$ |
| Ethansulfonsäure | $CH_3CH_2SO_3H$ |
| Propansulfonsäure | $CH_3(CH_2)_2SO_3H$ |
| Butansulfonsäure | $CH_3(CH_2)_3SO_3H$ |
| Pentansulfonsäure | $CH_3(CH_2)_4SO_3H$ |
| Hexansulfonsäure | $CH_3(CH_2)_5SO_3H$ |
| Vinylsulfonsäure | $CH_2=CHSO_3H$ |
| Methallylsulfonsäure | $CH_2=C(CH_3)-CH_2-SO_3H$ |
| Benzolsulfonsäure | $C_6H_5SO_3H$ |
| Toluolsulfonsäure | $CH_3C_6H_4SO_3H$ |

Beispiele für Sulfon- oder Carbonsäurederivate:

Sulfon-/Carbonsäurehalogenide
Sulfon-/Carbonsäureester
Sulfon-/Carbonsäureanhydride
Sulfon-/Carbonsäuresalze.

Ein weiterer Gegenstand der Erfindung ist die Verwendung der erfindungsgemäßen Imide und ihrer Salze als grenzflächenaktive Wirkstoffe.

Aufgrund der hohen Grenzflächenaktivität der erfindungsgemäßen Imidverbindungen können die Imidverbindungen beispielsweise in den folgenden Anwendungsgebieten eingesetzt werden:

In elektrolytischen Prozessen (z.B. bei der galvanischen Verchromung, Verkupferung und Vernickelung, beim Anodisieren und bei der elektrolytischen Entfettung) können die erfindungsgemäßen Verbindungen zur Sprühnebelunterdrückung und zur Verhinderung von Austragsverlusten zugesetzt werden.

In nicht elektrolytischen Badprozessen (z.B. bei der chemischen Verkupferung oder Vernickelung, bei der chemischen Entfettung oder Entrostung, beim Ätzen oder Gravieren, beim Glanztauchen, beim Beizen, Brünieren oder Passivieren, beim Eloxieren oder beim Entmetallisieren) können die erfindungsgemäßen Verbindungen als Sprühnebelunterdrücker und Reinigungshilfsmittel zugesetzt werden.

In Reinigungs- und Pflegemitteln (wie z.B. in Glas-, Herd-, Auto-, Gebäude-, Fassaden- oder Metalloberflächenreinigungsmitteln, in Fleckentfernern, in Shampoos, in Polituren für Möbel, Autos usw., in Selbstglanzemulsionen oder in Wachsen) können die erfindungsgemäßen Verbindungen als Verlaufs-, Spreit- und Netzmittel sowie zur Unterstützung der Eigenschaften, die die Wiederanschmutzung verhindern, zugesetzt werden.

Die erfindungsgemäßen Verbindungen können als solche oder in Formulierungen als Antibeschlagmittel oder Anlaufschutz (z.B. für Gläser, Metalle oder Kunststoffe) eingesetzt werden.

Die erfindungsgemäßen Verbindungen können als solche oder in Formulierungen als Korrosionsinhibitoren oder Korrosionsschutzüberzüge (z.B. in Polymerisationsreaktionen, für Füllstoffe, Fasern, Salze oder magnetische Feststoffe, in Lacken oder Blutersatzstoffen) eingesetzt werden.

Aufgrund ihrer Neigung, gasdichte Sperrschichten auszubilden und damit das Verdampfen oder Verdunsten von Flüssigkeiten zu verhindern, eignen sich die erfindungsgemäßen Verbindungen auch als Zusätze zu Feuerlöschmitteln.

Die erfindungsgemäßen Verbindungen können als Formtrennmittel eingesetzt werden.

In Farben und Lacken bewirkt ein Zusatz der erfindungsgemäßen Verbindungen eine Verbesserung der Verlaufs-, Netz- und Haftungseigenschaften. Außerdem verhindern sie durch Förderung der Entlüftung die Bildung von Oberflächendefekten (wie z.B. Kraterbildung oder Kantenflucht). Ferner wird durch ihren Zusatz eine bessere Verteilung der Pigmente erreicht. Von besonderem Vorteil ist die nicht schaumstabilisierende Wirkung der erfindungsgemäßen Verbindungen in Rezepturen bei der Herstellung von wasserverdünnbaren Lacken.

Die Neigung der erfindungsgemäßen Verbindungen, hydrophobe und oleophobe Sperrschichten auszubilden, macht einen Einsatz in Bautenschutzmitteln (z.B. zur Isolierung gegen Umwelteinflüsse) möglich.

Die erfindungsgemäßen Verbindungen können als Fließ- oder Gleitmittel (z.B. in Mineralerzen oder -salzen, auf Magnetbändern oder in Baustoffen) eingesetzt werden.

Die erfindungsgemäßen Verbindungen eignen sich als Schmiermittel, Schneidölzusätze oder Hydrauliköle.

Die erfindungsgemäßen Verbindungen können als Bohrhilfsmittel (z.B. Leistungssteigerung bei Ölbohrungen) eingesetzt werden.

In Fotochemikalien oder bei der Filmherstellung können die erfindungsgemäßen Verbindungen (z.B. als Netzmittel oder Antistatikum) eingesetzt werden.

In Pflanzenschutzmitteln können die erfindungsgemäßen Verbindungen (z.B. als Netzund Verlaufmittel) eingesetzt werden.

Ein Zusatz der erfindungsgemäßen Verbindungen zu Ausrüstungsmitteln für Textilien, Leder oder Papier kann beispielsweise die Benetzung oder das Eindringen des Ausrüstungsmittels fördern, zu einer Entschäumung führen oder dessen Hydrophob-/Oleophobwirkung unterstützen.

Die Erfindung soll anhand folgender Beispiele näher erläutert werden.

## Beispiele

### Beispiel 1

In einer Dreihalskolben-Rührapparatur werden 0,3 mol (273,9 g) Polyether der Formel $H_2N(CH_2CH_2O)_{19,6}C_4H_9$ und 0,3 mol (30,3 g) Triethylamin auf 50°C erhitzt, 0,3 mol (150,6 g) Perfluoroctylsulfonylfluorid über 15 min zugegeben und 4 h 30 min am Rückfluß gerührt.

Nach dem Abkühlen wird mit weiteren 0,3 mol (30,3 g) Triethylamin versetzt, auf 50°C erhitzt, 0,3 mol (90,6 g) Perfluorbutylsulfonylfluorid über 15 min zugegeben und 2 h am Rückfluß gerührt.

Die Ausbeute der gewünschten N-Perfluorbutylsulfon-N-perfluoroctylsulfon-imid-Verbindung beträgt 491 g (entsprechend 98 % der Theorie). Die Oberflächenspannung einer 0,1-%igen wäßrigen Lösung beträgt 28,1 mN/m, die einer entsprechenden einprozentigen Lösung 26,0 mN/m (gemessen mit einem Ringtensiometer der Fa. Lauda).

### Beispiel 2

In einer Dreihalskolben-Rührapparatur werden 0,3 mol (273,9 g) Polyether der Formel $H_2N(CH_2CH_2O)_{19,6}C_4H_9$ und 0,6 mol (60,6 g) Triethylamin auf 50°C erhitzt, 0,6 mol (181,2 g) Perfluorbutylsulfonylfluorid

über 15 min zugegeben und 10 h am Rückfluß gerührt.

Die Ausbeute der gewünschten Bis-perfluorbutylsulfon-imid-Verbindung beträgt 421 g (entsprechend 95 % der Theorie). Die Oberflächenspannung einer 0,1-%igen wäßrigen Lösung beträgt 36,6 mN/m, die einer entsprechenden einprozentigen Lösung 26,5 mN/m (gemessen mit einem Ringtensiometer der Fa. Lauda).

**Beispiel 3**

In einer Dreihalskolben-Rührapparatur werden 0,3 mol (273,9 g) Polyether der Formel $H_2N(CH_2CH_2O)_{19,6}C_4H_9$ und 0,3 mol (30,3 g) Triethylamin auf 50°C erhitzt, 0,3 mol (90,6 g) Perfluorbutylsulfonylfluorid über 15 min zugegeben und 2 h am Rückfluß gerührt.

Nach dem Abkühlen wird mit weiteren 0,3 mol (30,3 g) Triethylamin versetzt, auf 50°C erhitzt, 0,3 mol (58,8 g) Octylsulfonylfluorid über 15 min zugegeben und 24 h am Rückfluß gerührt.

Nach dem Abkühlen wird mit 150 ml Wasser gewaschen und bei 40°C und 50 mbar getrocknet. Die Ausbeute der gewünschten N-Perfluorbutylsulfon-N-octylsulfonimid-Verbindung beträgt 276 g (entsprechend 67 % der Theorie). Die Oberflächenspannung einer 0,1-%igen wäßrigen Lösung beträgt 41,6 mN/m, die einer entsprechenden einprozentigen Lösung 30,7 mN/m (gemessen mit einem Ringtensiometer der Fa. Lauda).

## Patentansprüche

1. Fluoralkyl- und/oder fluorarylgruppenhaltige Imidverbindungen der allgemeinen Formel (I):

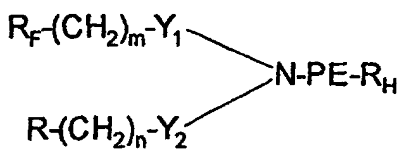

(I) ,

worin

$R_F$ ein linearer oder verzweigter Fluoralkylrest mit 1 bis 18 Kohlenstoffatomen, ein Fluorarylrest mit 6 bis 12 Kohlenstoffatomen ein gemischter Fluoralkylarylrest mit 7 bis 18 Kohlenstoffatomen oder ein fluorierter Mono- oder Polyetherrest mit 2 bis 18 Kohlenstoffatomen ist,

R ein linearer oder verzweigter Alkylrest mit 1 bis 24 Kohlenstoffatomen, ein Arylrest mit 6 bis 12 Kohlenstoffatomen oder ein gemischter Alkylarylrest mit 7 bis 24 Kohlenstoffatomen, wobei die Kohlenstoffkette auch durch Sauerstoff-, Stickstoff- oder Schwefelatome unterbrochen sein kann, oder ein weiterer wie oben definierter Rest $R_F$ ist, wobei die beiden $R_F$-Reste gleich oder unterschiedlich sein können,

$Y_1$ und $Y_2$ unabhängig voneinander eine

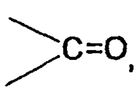 , 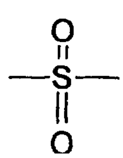 , 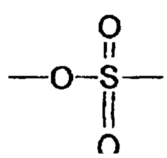 oder 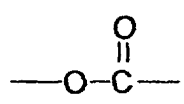 Gruppe

darstellen,

m eine ganze Zahl zwischen 0 und 6 darstellt,

n eine ganze Zahl zwischen 0 und 6 darstellt und

PE eine Polyetherkette bestehend aus 5 bis 50 Ethylenoxid- oder 5 bis 50 Propylenoxid- oder einem Gemisch

aus 5 bis 50 Ethylenoxid- und Propylenoxideinheiten darstellt und

$R_H$ ein Wasserstoffatom oder ein linearer oder verzweigter Alkylrest mit 1 bis 10 Kohlenstoffatomen ist.

**2.** Fluoralkyl- und/oder fluorarylgruppenhaltige Imidverbindungen gemäß Anspruch 1, **dadurch gekennzeichnet, daß** $R_F$ ein linearer oder verzweigter Fluoralkylrest mit 3 bis 10 Kohlenstoffatomen bzw. ein Fluorarylrest mit 6 bis 12 Kohlenstoffatomen ist.

**3.** Fluoralkyl- und/oder fluorarylgruppenhaltige Imidverbindungen gemäß Anspruch 1 oder 2, **dadurch gekennzeichnet, daß** $R_F$ für einen linearen oder verzweigten Perfluoralkylrest mit 3 bis 10 Kohlenstoffatomen bzw. für einen Perfluorarylrest mit 6 bis 12 Kohlenstoffatomen steht.

**4.** Imidverbindungen gemäß einem oder mehreren der Ansprüche 1 bis 3, **dadurch gekennzeichnet, daß** R für einen linearen oder verzweigten Alkylrest mit 6 bis 14 Kohlenstoffatomen, einen Arylrest mit 6 bis 12 Kohlenstoffatomen, einen gemischten Alkylarylrest mit 7 bis 14 Kohlenstoffatomen oder einen linearen oder verzweigten Perfluoralkylrest mit 3 bis 10 Kohlenstoffatomen steht.

**5.** Imidverbindungen gemäß einem oder mehreren der Ansprüche 1 bis 4, **dadurch gekennzeichnet, daß** $Y_1$ und $Y_2$ unabhängig voneinander für eine

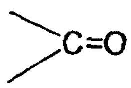 oder 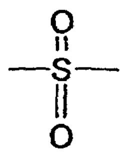 Gruppe

stehen.

**6.** Imidverbindungen gemäß einem oder mehreren der Ansprüche 1 bis 5, **dadurch gekennzeichnet, daß** m und n 0 sind.

**7.** Imidverbindungen gemäß einem oder mehreren der Ansprüche 1 bis 6, **dadurch gekennzeichnet, daß** $R_H$ für lineare Alkylreste mit 2 bis 4 Kohlenstoffatomen steht.

**8.** Verfahren zur Herstellung der fluoralkyl- und/oder fluorarylgruppenhaltigen Imidverbindungen gemäß den Ansprüchen 1 bis 7, **dadurch gekennzeichnet, daß** eine fluoralkyl- oder fluorarylgruppenhaltige Verbindung der Formel (II)

$$R_F\text{-}(CH_2)_m\text{-}Y_1\text{-}A_1$$

wobei

$R_F$, m, $Y_1$ dieselbe Bedeutung wie oben haben und $A_1$ eine reaktive Abgangsgruppe, wie z.B. ein Halogenatom, eine Hydroxy-, eine Alkoxyoder eine Carboxygruppe, darstellt

mit einem Amin der Formel (III)

$$H_2N\text{-}PE\text{-}R_H$$

wobei

PE und $R_H$ dieselbe Bedeutung wie oben haben, unter Zugabe von tertiärem Amin umgesetzt wird und das Reaktionsprodukt anschließend mit einer Verbindung der Formel (IV)

$$R - (CH_2)_n - Y_2 - A_2$$

wobei

R, n, und $Y_2$ dieselbe Bedeutung wie oben haben und $A_2$ dieselbe Bedeutung wie $A_1$ hat, unter Zugabe von tertiärem Amin umgesetzt wird.

**9.** Verwendung der fluoraryl- und/oder fluoralkylgruppenhaltigen Imidverbindungen gemäß den Ansprüchen 1 bis 7 als grenzflächenaktive Mittel.

**Claims**

**1.** Imide compounds containing fluoroalkyl and/or fluoroaryl groups, which compounds are of the general formula (I):

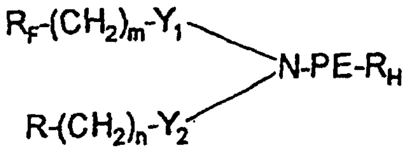 (I) ,

in which

$R_F$ is a linear or branched fluoroalkyl residue with 1 to 18 carbon atoms, a fluoroaryl residue with 6 to 12 carbon atoms, a mixed fluoroalkylaryl residue with 7 to 18 carbon atoms or a fluorinated mono or polyether residue with 2 to 18 carbon atoms,

R is a linear or branched alkyl residue with 1 to 24 carbon atoms, an aryl residue with 6 to 12 carbon atoms or a mixed alkylaryl residue with 7 to 24 carbon atoms, wherein the carbon chain may also be interrupted by oxygen, nitrogen or sulfur atoms, or a further residue $R_F$ as defined above, wherein the two $R_F$ residues may be identical or different,

$Y_1$ and $Y_2$ mutually independently represent a

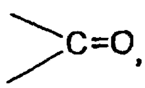, 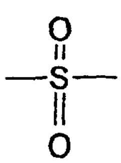 , 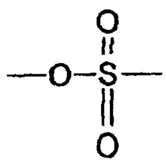 or 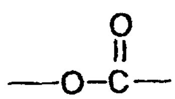 group

m represents an integer between 0 and 6,

n represents an integer between 0 and 6 and

PE represents a polyether chain consisting of 5 to 50 ethylene oxide units or 5 to 50 propylene oxide units or a mixture of 5 to 50 ethylene oxide and propylene oxide units and

$R_H$ is a hydrogen atom or a linear or branched alkyl residue with 1 to 10 carbon atoms.

**2.** Imide compounds containing fluoroalkyl and/or fluoroaryl groups according to claim 1, **characterised in that** $R_F$ is a linear of branched fluoroalkyl residue with 3 to 10 carbon atoms or a fluoroaryl residue with 6 to 12 carbon atoms.

**3.** Imide compounds containing fluoroalkyl and/or fluoroaryl groups according to claim 1 or 2, **characterised in that**

$R_F$ denotes a linear or branched perfluoroalkyl residue with 3 to 10 carbon atoms or for a perfluoroaryl residue with 6 to 12 carbon atoms.

**4.** Imide compounds according to one or more of claims 1 to 3, **characterised in that** R denotes a linear or branched alkyl residue with 6 to 14 carbon atoms, an aryl residue with 6 to 12 carbon atoms, a mixed alkylaryl residue with 7 to 14 carbon atoms or a linear or branched perfluoroalkyl residue with 3 to 10 carbon atoms.

**5.** Imide compounds according to one or more of claims 1 to 4, **characterised in that** $Y_1$ and $Y_2$ mutually independently denote a

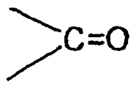

or

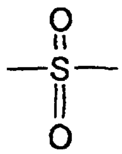

group

**6.** Imide compounds according to one or more of claims 1 to 5, **characterised in that** m and n are 0.

**7.** Imide compounds according to one or more of claims 1 to 6, **characterised in that** $R_H$ denotes a linear alkyl residue with 2 to 4 carbon atoms.

**8.** A process for the production of the imide compounds containing fluoroalkyl and/or fluoroaryl groups according to claims 1 to 7, **characterised in that** a compound of the formula (II) containing fluoroalkyl or fluoroaryl groups

$$R_F\text{-}(CH_2)_m\text{-}Y_1\text{-}A_1$$

wherein

$R_F$, m, $Y_1$ have the same meaning as above and $A_1$ represents a reactive leaving group, such as for example a halogen atom, a hydroxy, alkoxy or carboxy group

is reacted with an amine of the formula (III)

$$H_2N\text{-}PE\text{-}R_H$$

wherein
PE and $R_H$ have the same meaning as above, with the addition of tertiary amine, and the reaction product is subsequently reacted with a compound of the formula (IV)

$$R\text{-}(CH_2)_n\text{-}Y_2\text{-}A_2$$

wherein
R, n, and $Y_2$ have the same meaning as above and $A_2$ has the same meaning as $A_1$, with the addition of tertiary amine.

**9.** Use of the imide compounds containing fluoroaryl and/or fluoroalkyl groups according to claims 1 to 7 as surface-active agents.

## Revendications

**1.** Imides à groupes fluoralkyle et/ou fluoraryle de formule générale (I) :

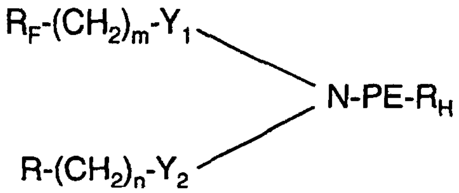

(I),

dans laquelle

- $R_F$ représente un groupe fluoralkyle à chaîne droite ou ramifiée en $C_1$-$C_{18}$, un groupe fluoraryle en $C_6$-$C_{12}$, un groupe mixte fluoralkylaryle en $C_7$-$C_{18}$ ou un groupe mono- ou poly-éther fluoré en $C_2$-$C_{18}$,
- R représente un groupe alkyle à chaîne droite ou ramifiée en $C_1$-$C_{24}$, un groupe aryle en $C_6$-$C_{12}$ ou un groupe mixte alkylaryle en $C_7$-$C_{24}$, la chaîne carbonée pouvant également être interrompue par des atomes d'oxygène, d'azote ou de soufre, ou un autre groupe $R_F$ tel que défini ci-dessus, les deux groupes $R_F$ pouvant être identiques ou différents,
- $Y_1$ et $Y_2$ représentent chacun, indépendamment l'un de l'autre un groupe

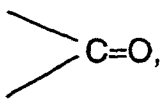,

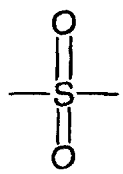,

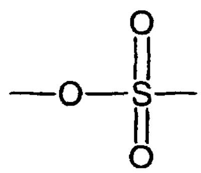

ou

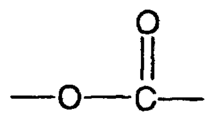

- m est un nombre entier allant de 0 à 6,
- n est un nombre entier allant de 0 à 6,
- PE représente une chaîne de polyéther consistant en 5 à 50 motifs d'oxyde d'éthylène ou 5 à 50 motifs d'oxyde de propylène ou en un mélange de 5 à 50 motifs d'oxyde d'éthylène et d'oxyde de propylène et
- $R_H$ représente un atome d'hydrogène ou un groupe alkyle à chaîne droite ou ramifiée en $C_1$-$C_{10}$.

**2.** Imides à groupes fluoralkyle et/ou fluoraryle selon la revendication 1, **caractérisés en ce que** $R_F$ représente un groupe fluoralkyle à chaîne droite ou ramifiée en $C_3$-$C_{10}$ ou un groupe fluoraryle en $C_6$-$C_{12}$.

**3.** Imides à groupes fluoralkyle et/ou fluoraryle selon la revendication 1 ou 2, **caractérisés en ce que** $R_F$ représente un groupe perfluoralkyle à chaîne droite ou ramifiée en $C_3$-$C_{10}$ ou un groupe perfluoraryle en $C_6$-$C_{12}$.

**4.** Imides selon une ou plusieurs des revendications 1 à 3, **caractérisés en ce que** R représente un groupe alkyle à chaîne droite ou ramifiée en $C_6$-$C_{14}$, un groupe aryle en $C_6$-$C_{12}$, un groupe mixte alkylaryle en $C_7$-$C_{14}$ ou un groupe perfluoralkyle à chaîne droite ou ramifiée en $C_3$-$C_{10}$.

**5.** Imides selon une ou plusieurs des revendications 1 à 4, **caractérisés en ce que** $Y_1$ et $Y_2$ représentent chacun, indépendamment l'un de l'autre, un groupe

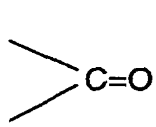

ou

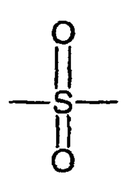.

**6.** Imides selon une ou plusieurs des revendications 1 à 5, **caractérisés en ce que** m et n sont égaux à 0.

**7.** Imides selon une ou plusieurs des revendications 1 à 6, **caractérisés en ce que** $R_H$ représente un groupe alkyle à chaîne droite en $C_2$-$C_4$.

**8.** Procédé pour la préparation des imides à groupes fluoralkyle et/ou fluoraryle selon les revendications 1 à 7, **caractérisé en ce que** l'on fait réagir un composé à groupes fluoralkyle ou fluoraryle de formule (II)

$$R_F\text{-}(CH_2)_m\text{-}Y_1\text{-}A_1$$

dans laquelle

$R_F$, m, $Y_1$ ont les significations indiquées ci-dessus et A1 représente un substituant réactif éliminable, par exemple un atome d'halogène, un groupe hydroxy, alkoxy ou carboxy,

avec une amine de formule (III)

$$H_2N\text{-}PE\text{-}R_H$$

dans laquelle

PE et $R_H$ ont les significations indiquées ci-dessus, en présence d'une amine tertiaire, puis on fait réagir le produit obtenu avec un composé de formule (IV)

$$R\text{-}(CH_2)_n\text{-}Y_2\text{-}A_2$$

dans laquelle

R, n et $Y_2$ ont les significations indiquées ci-dessus et $A_2$ a les mêmes significations que $A_1$, en présence d'une amine tertiaire.

**9.** Utilisation des imides à groupes fluoraryle et/ou fluoralkyle selon les revendications 1 à 7 en tant qu'agents tensioactifs.